# EUROPEAN PATENT APPLICATION

(11) **EP 2 722 037 A1**
(43) Date of publication of application: **23.04.2014**
(21) Application number: 13189178.0
(22) Date of filing: 17.10.2013
(51) Int. Cl.: A61K 9/20, A61K 31/4365

(54) **Prasugrel formulations**

(30) Priority: 19.10.2012 TR 201212084
(71) Applicant: Sanovel Ilac Sanayi ve Ticaret A.S., 34460 Istanbul (TR)
(72) Inventor: Cifter, Ümit, 34460 Istanbul (TR); Mutlu, Onur, 34460 Istanbul (TR); Toker, Özlem, 34460 Istanbul (TR)
(74) Representative: Sevinç, Erkan

(57) **Abstract**

The present invention relates to formulations comprising prasugrel free base. More specifically, the present invention relates to prasugrel formulations, which are resistant against environmental and physical conditions and are stable under production and storage conditions.

## Description

### Field of Invention

The present invention relates to pharmaceutical formulations comprising prasugrel free base. More specifically, the present invention relates to prasugrel formulations, which are resistant against environmental and physical conditions and are stable under production and storage conditions.

### Background of Invention

Prasugrel hydrochloride is a member of the thienopyridine class and inhibits the activation and aggregation of platelets by means of P2Y12 and ADP receptors. Its chemical name is 2-acetoxy-5-(α-cyclopropylcarbonyl-2-fluorobenzyl)-4,5,6,7-tetrahydrothieno [3,2-c]pyridine hydrochloride, with the chemical structure illustrated below in Formula 1.

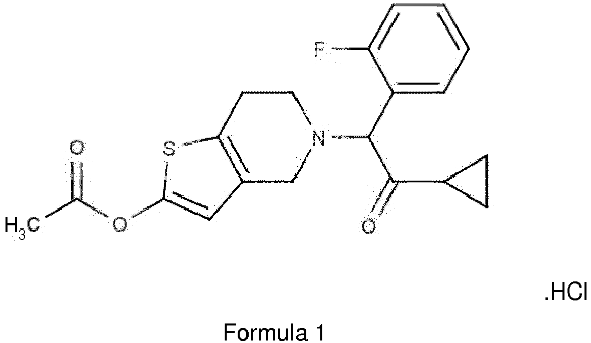

Prasugrel hydrochloride is a white and solid. It is well soluble at pH 2, weakly soluble at pH 3 to 4, and is substantially insoluble at pH 6 to 7.5.

Prasugrel hydrochloride is marketed under the trademark Effient^{®} in 5 or 10 mg dosages. It is initially administered in a dosage amount of 60 mg as a loading dose. It is used in preventing or treating thrombosis and cardiovascular diseases.

Searching the patent literature reveals various patents in relation to prasugrel.

The patent application EP1298132 - discloses hydrochloride salt of prasugrel, a method for preparing this salt, and its use for thrombosis and embolism.

The patent application EP1728794 discloses maleate salt prasugrel, a method for preparing this salt, and its use for thrombosis and embolism.

The patent application WO2006135605 discloses a formulation containing prasugrel hydrochloride, packaged in a blister package which is air- and moisture-impermeable, and is inert against gasd..

Patent application WO2008073759 discloses a tablet or capsule formulation containing prasugrel, packaged in an air- and moisture-impermeable blister under a gas with positive fluidity pressure in order to reduce the amount of oxygen and humidity generated during packaging.

Stability-related problems occur in many active agents, including prasugrel, under the influence of environmental and physical conditions. However, prasugrel is an active agent that is highly-susceptible to air and humidity. When prasugrel is exposed to air and humidity, it degrades structurally and develops chemical behavioral changes. As a result of this, two main problems emerge. The first problem is that the stability of the products developed is not at a desired level and the shelf life thereof is shortened. The second problem is that prasugrel reacts with the excipients employed in developing formulations containing prasugrel. This fact causes impurities to occur in the formulation and leads to incorporation of undesired components into the formulation. In addition to these, measures should be taken while preparing, packaging, and storing finished dosage forms comprising prasugrel.

Therefore, a novelty is required in the art of prasugrel formulations used for preventing and treating thrombosis and cardiovascular diseases, which would overcome the aforesaid drawbacks.

### Object and Brief Description of Invention

The present invention provides a prasugrel formulation, eliminating all aforesaid problems and bringing additional advantages to the relevant prior art.

Accordingly, the main object of the present invention is to provide a prasugrel formulation which is resistant against environmental and physical conditions.

A further object of the present invention is to provide a prasugrel formulation which is stable under production and storage conditions and has a high bioavailability.

Another object of the present invention is to provide a prasugrel formulation, which is both resistant against environmental and physical conditions, and is stable under production and storage conditions, as well as has a high bioavailability.

A further object of the present invention is to provide a simple, cost-effective, and time-saving process for preparing a prasugrel formulation.

Another object of the present invention is to increase the stability of the finished dosage forms of the pharmaceutical formulation under production, packaging, and storage conditions by using suitable excipients during the preparation of the formulation.

In order to achieve the objects referred to above and to be described in the following detailed description, a pharmaceutical formulation comprising prasugrel free base is developed.

In a preferred embodiment of the present invention, the novelty is realized by providing a pharmaceutical formulation which comprises prasugrel free base.

In another preferred embodiment of the present invention, said formulation comprises prasugrel free base and at least one excipient.

In a further preferred embodiment of the present invention, one or more pharmaceutically acceptable excipient(s) is/are selected from a group comprising fillers, disintegrants, binders, lubricants, glidants, sweeteners, aromatic agents, preservatives, coloring agents, buffering agents, solvents, surface active agents and the mixtures thereof.

Another embodiment of the present invention provides a method for preparing a pharmaceutical formulation comprising prasugrel free base, this method comprising the steps of:
a. mixing a part of all excipients except for the active agent in a container (mixture A), and the remaining part thereof in a separate container (mixture B),
b. mixing prasugrel free base with mixture A from the excipient mixtures obtained above in step (a) and then mixing the resulting mixture with mixture B,
c. converting the mixture obtained in step (b) into a suitable dosage form and packaging the same.

Following step (a) described above for preparing a formulation according to the present invention, and depending on the type of the dosage form of the finished product to be obtained, prasugrel free base used as the active agent of the formulation is preferably brought to a suitable particle size before it is mixed with the mixture of excipients obtained in step (a).

Accordingly, the mean particle size of prasugrel free base contained in the formulation according to the present invention is preferably between 1 µm and 100 µm and more preferably between 5 µm and 20 µm.

### Detailed Description of Invention

In pharmaceutical formulations comprising prasugrel as an active agent, the salt of prasugrel, particularly the hydrochloride or maleate salt, is used. For instance, in the commercially available product Effient®, the active agent is prasugrel hydrochloride.

However, as a result of studies made, it was surprisingly found that pharmaceutical formulations comprising prasugrel free base is more resistant against environmental and physical conditions as compared to pharmaceutical formulations comprising other forms, particularly the hydrochloride and maleate salts, of prasugrel.

With this invention, prasugrel formulations is prepared which are resistant against environmental and physical conditions, and have an improved stability under production and storage conditions, as well as have a high bioavailability.

The pharmaceutical formulation according to the present invention comprises prasugrel free base as an active agent. In other words, the formulation according to the present invention is substantially free of other forms of prasugrel, except for the free base of prasugrel.

In a preferred embodiment of the present invention, said formulation may further comprise other forms of prasugrel as an active agent beside prasugrel free base. The total amount of other forms of prasugrel in addition to the free base thereof as an active agent in said pharmaceutical formulation is at most 1% based on the total weight of all prasugrel forms. Accordingly, prasugrel free base contained in the formulation as an active agent has a percentage purity preferably of at least 99% and more preferably of at least 99.5%.

Other forms of prasugrel contained in the formulation according to the present invention in addition to prasugrel free base are preferably selected from a group comprising, but not limited to, prasugrel isomers, enantiomers, racemates, salts (preferably hydrochloride and maleate salts), the solvates and hydrates of these salts, and the mixtures thereof.

In a preferred embodiment of the present invention, the amount of prasugrel free base is from 1% to 90%, preferably from 5% to 70%, and more preferably from 10% to 60% of the total weight of the formulation.

According to the presen invention, the mean particle size of prasugrel free base contained in the formulation is preferably between 1 µm and 100 µm and more preferably between 5 µm and 20 µm.

On the other hand, the particle size of prasugrel free base used in the pharmaceutical formulation is preferably measured by means of laser diffraction method. More specifically, the particle size of prasugrel free base is measured using a dry dispersion method making use of air as a dispensing agent on a "Malvern Mastersizer 2000 Particle Size Analyzer". The term of "mean particle size" refers to the d_{0.5} particle size by volume obtained using the measurement method described above. The d_{0.5} particle size by volume defines the maximum particle size of less than 50% by volume of the particles.

The pharmaceutical formulation according to the present invention can be administered via oral, parenteral, ocular, nasal, buccal, sublingual, transdermal, transmucozal, intramuscular, rectal, topical, or inhalation route, wherein the preferred route of administration is the oral route.

The formulation according to the present invention may be in the form of a tablet, dragee, capsule, caplet, orally-disintegrating tablet, film-coated tablet, enteric tablet, buccal tablet, sublingual tablet, chewable tablet, effervescent tablet, slow-release tablet, rapid-release tablet, modified-release tablet, delayed-release tablet, prolonged-release, controlled-release tablet, sachet, granule, pilule, powder, pellet, suppository, pastille and similar solid oral dosage forms, or syrup, elixir, solution, suspension, drop (concentrated solution), potion, emulsion, ampoule, or similar liquid oral dosage forms. The preferred dosage form according to the present invention is the solid dosage form,preferably a tablet form.

The pharmaceutical formulations according to the present invention may also comprise one or more pharmaceutically acceptable excipient(s). Pharmaceutically acceptable excipients comprise, but are not limited to, fillers, disintegrants, binders, lubricants, glidants, sweeteners, aromatic agents, preservatives, coloring agents, and the mixtures thereof. Excipients widely used in dosage forms and the examples thereof are given below.

In a preferred embodiment of the present invention, said fillers comprise, but are not limited to, polysaccharides such as mannitol, spray-dried mannitol; microcrystalline cellulose, pullulan; dibasic calcium phosphate dihydrate, lactose, sugars, sorbitol; mixture of microcrystalline cellulose and guar gum (Avicel CE-15); mixture of mannitol, polyplasdone and syloid (Pharmaburst); mixture of mannitol, crospovidone and polyvinyl acetate (Ludiflash); isomalt, Panexcea, F-Melt, sucrose, calcium salts and similar inorganic salts; heavy magnesium carbonate, and the mixtures thereof.

The percentage amount of the fillers contained in a formulation according to the present invention is between 1% and 90%, preferably between 5% and 85%, and more preferably between 10% and 75%, based on the total weight of the formulation.

In a preferred embodiment of the present invention, said disintegrants comprise, but are not limited to, at least one or a mixture of sodium starch glycolate, croscarmellose sodium, crospovidone, sodium alginate, gums, starch, and magnesium aluminum silicate.

The inventor has surprisingly found that when the filler and disintegrant are contained in the formulation at a particular weight ratio, namely when the ratio by weight of the filler to the disintegrant is kept between 80:1 and 1:20, preferably between 50:1 and 1:10, and more preferably between 30:1 and 1:5, the finished dosage forms of the formulation became more stable under production, packaging, and storage conditions.

In a preferred embodiment of the present invention, said binders comprise, but are not limited to, polyvinylpyrrolidone (povidone), gelatin, sugars, glucose, natural gums, gums, synthetic celluloses, polymethacrylate, hydroxypropyl methylcellulose, hydroxypropyl cellulose, carboxymethyl cellulose, methyl cellulose, and other cellulose derivatives, and the mixtures thereof.

In a preferred embodiment of the present invention, said lubricants comprise, but are not limited to, sodium stearyl fumarate, magnesium stearate, polyethylene glycol, stearic acid, metal stearates, boric acid, sodium chloride benzoate and acetate, hydrogenated castor oil, sodium or magnesium lauryl sulfate, and the mixtures thereof.

In a preferred embodiment of the present invention, said glidants comprise, but are not limited to, colloidal silicon dioxide, talk, aluminium silicate, and the mixtures thereof.

In a preferred embodiment of the present invention, said sweeteners comprise, but are not limited to, aspartame, sucralose, saccharine; glucose, lactose, fructose and other sugars, and mannitol, sorbitol, xylitol, erythritol and other sugar alcohols, and the mixtures thereof.

In a preferred embodiment of the present invention, said aromatic agents comprise, but are not limited to, menthol, mint, cinnamon, chocolate, vanillin, and fruit extracts such as of cherry, orange, strawberry, grape, and the mixtures thereof.

The following additional excipients can also be used in the pharmaceutical formulation according to the present invention:

Coating agents, including, but not limited to, polymers such as hydroxypropyl methylcellulose, polyethylene glycol, polyvinylpyrrolidone, polyvinylpyrrolidone-vinyl acetate copolymer (PVP-VA), pullulan, and all kinds of Opadry, as well as pigments, dyes, titanium dioxide and iron oxide, talk.

Colorants, including, but not limited to, Food, Drug, and Cosmetic (FD&C) dyes (e.g. FD&C blue, FD&C green, FD&C red, FD&C yellow, FD&C lake), ponceau, indigo Drug & Cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow, and the mixtures thereof.

Preservatives, including, but not limited to, methylparaben and propylparaben and the salts thereof (e.g. sodium or potassium salts), sodium benzoate, citric acid, benzoic acid, butylated hydroxytoluene and butylated hydroxyanisole, and the mixtures thereof.

The process used for preparing a pharmaceutical formulation according to the present invention comprises the steps of, first, mixing a part of all excipients except for the active agent in a container (mixture A), and the remaining part thereof in a separate container (mixture B); mixing prasugrel free base with mixture A from the excipient mixtures obtained above in step (a) and then mixing the resulting mixture with mixture B; converting the resulting mixture into a suitable dosage form and packaging the same.

In a preferred embodiment of the present invention, a process for use in the preparation of a pharmaceutical formulation according to the present invention comprises the steps of, first, mixing the filler and the disintegrant among all excipients except for the active agent in a container (mixture A), and mixing the other excipients in a separate container (mixture B); mixing prasugrel free base with mixture A from the excipient mixtures obtained in step (a) and then mixing the resulting mixture with mixture B; converting the resulting mixture into a suitable dosage form and packaging the same.

Accordingly, the pharmaceutical formulation according to the present invention comprising prasugrel free base as an active agent is unexpectedly resistant against environmental and physical conditions and both shows a substantially improved stability under production and storage conditions, and has a high bioavailability. Using prasugrel free base which purified from the other forms of prasugrel as the active agent brings the stability of the formulation to a desired level. Additionally, above-mentioned process for preparing the formulation, helps the stability of finished dosage forms of the formulation under production, packaging, and storage conditions to be increased as well.

The present invention is used for treating thrombosis and cardiovascular diseases.

These formulations can be used in the content of medicaments which are efficient in preventing or treating thrombosis and cardiovascular diseases.

The present invention is described in the following examples in more details. These examples are not limiting the scope of the present invention and is to be considered under the light of the foregoing detailed description. It is obvious that those skilled in the relevant art can produce several embodiments under the light of the foregoing disclosures by making many adaptations to both the materials and the methods, without going beyond the scope of the present invention.

### Example 1

| **Formulation Content** | **Amount (%)** |
|---|---|
| *Core tablet* | |
| Prasugrel free base | 25%-45% |
| Lactose | 30%-50% |
| Microcrystalline cellulose | 1%-10% |
| Polyethylene glycol | 1%-15% |
| Hydroxypropyl cellulose | 1%-20% |
| Hydrogenated castor oil | 1%-5% |

The pharmaceutical formulation mentioned above is prepared as following: mannitol and microcrystalline cellulose are mixed in a container (mixture A); hydroxypropyl cellulose, polyethylene glycol and hydrogenated castor oil are mixed in a separate container (mixture B); prasugrel free base is first mixed with mixture A and then the resulting mixture is mixed with mixture B; the mixture is compressed into tablets. Finally, the compressed tablets are preferably coated with a film coating.

### Example 2

| **Formulation Content** | **Amount (%)** |
|---|---|
| *Core tablet* | |
| Prasugrel free base | 1%-50% |
| Mannitol | 1%-20% |
| Microcrystalline cellulose | 1%-90% |
| Polyvinylpyrrolidone | 1%-20% |
| Croscarmellose sodium | 2%-15% |
| Colloidal silicon dioxide | 0.1%-5% |
| Magnesium stearate | 0.2%-5% |

The pharmaceutical formulation mentioned above is prepared as following: mannitol, microcrystalline cellulose and croscarmellose sodium are mixed in a container (mixture A); polyvinylpyrrolidone, colloidal silicon dioxide, and magnesium stearate are mixed in another container (mixture B); prasugrel free base is first mixed with mixture A and then the resulting mixture is mixed with mixture B; the mixture is compressed into tablets. Finally, the compressed tablets are preferably coated with a film coating.

### Example 3

| **Formulation Content** | **Amount (%)** |
|---|---|
| *Core tablet* | |
| Prasugrel free base | 1%-60% |
| Pullulan | 1%-10% |
| Microcrystalline cellulose | 1%-80% |
| Polyvinylpyrrolidone | 1%-25% |
| Croscarmellose sodium | 2%-15% |
| Colloidal silicon dioxide | 0.1%-5% |
| Magnesium stearate | 0.2%-5% |

The pharmaceutical formulation mentioned above is prepared as following: pullulan, microcrystalline cellulose and croscarmellose sodium are mixed in a container (mixture A); polyvinylpyrrolidone, colloidal silicon dioxide, and magnesium stearate are mixed in another container (mixture B); prasugrel free base is first mixed with mixture A and then the resulting mixture is mixed with mixture B; the mixture is compressed into tablets. Finally, the compressed tablets are preferably coated with a film coating.

## Claims

1. A pharmaceutical formulation comprising prasugrel free base.

2. The pharmaceutical formulation according to Claim 1, further comprising preferably other forms of prasugrel beside the prasugrel free base.

3. The pharmaceutical formulation according to claims 1 and 2, wherein the percentage purity of prasugrel free base is at least 99%.

4. The pharmaceutical formulation according to claims 1 to 3, wherein the percentage purity of prasugrel free base is at least 99.5%.

5. The pharmaceutical formulation according to any of the preceding claims, wherein said other forms of prasugrel contained in the formulation beside the prasugrel free base are preferably selected from a group comprising prasugrel isomers, enantiomers, racemates, salts (preferably hydrochloride and maleate salts), the solvates and hydrates of these salts and the mixtures thereof.

6. The pharmaceutical formulation according to any of the preceding claims, wherein the amount of prasugrel free base is from 1% to 90%, preferably from 5% to 70%, and more preferably from 10% to 60% of the total weight of the formulation.

7. The pharmaceutical formulation according to any of the preceding claims, wherein the mean particle size of prasugrel free base contained in the formulation is between 1 and 100 µm and preferably between 5 and 20 µm.

8. The pharmaceutical formulation according to any of the preceding claims, wherein said formulation is administered via oral, parenteral, ocular, nasal, buccal, sublingual, transdermal, transmucosal, intramuscular, rectal, topical, or inhalation route.

9. The pharmaceutical formulation according to Claim 8, wherein said formulation is administered via the oral route.

10. The pharmaceutical formulation according to any of the preceding claims, wherein the formulation is preferably in a solid oral dosage form.

11. The pharmaceutical formulation according to Claim 10, wherein the formulation is in a solid oral dosage form selected from a group comprising a tablet, dragee, capsule, caplet, orally-disintegrating tablet, film-coated tablet, enteric tablet, buccal tablet, sublingual tablet, chewable tablet, effervescent tablet, slow-release tablet, rapid-release tablet, modified-release tablet, delayed-release tablet, prolonged-release, controlled-release tablet, sachet, granule, pilule, powder, pellet, suppository, pastille.

12. The pharmaceutical formulation according to Claim 11, wherein the formulation is preferably in a tablet form.

13. The pharmaceutical composition according to any of the preceding claims, further comprising at least one or more pharmaceutically acceptable excipient(s).

14. The pharmaceutical formulation according to Claim 13, wherein the pharmaceutically acceptable excipients comprise fillers, disintegrants, binders, lubricants, glidants, sweeteners, aromatic agents, preservatives, coloring agents, and the mixtures thereof.

15. The pharmaceutical formulation according to Claim 14, wherein said fillers comprise mannitol, spray-dried mannitol; microcrystalline cellulose, pullulan and similar polysaccharides; dibasic calcium phosphate dihydrate, lactose, sugars, sorbitol; mixture of microcrystalline cellulose and guar gum; mixture of mannitol, polyplasdone and syloid; mixture of mannitol, crospovidone and polyvinyl acetate; isomalt, Panexcea, F-Melt, sucrose, calcium salts and similar inorganic salts; heavy magnesium carbonate, and the mixtures thereof.

16. The pharmaceutical formulation according to claims 14 and 15, wherein the percentage amount of the fillers is between 1% and 90%, preferably between 5% and 85%, and more preferably between 10% and 75%, based on the total weight of the formulation.

17. The pharmaceutical formulation according to Claim 14, wherein said disintegrants comprise at least one or a mixture of sodium starch glycolate, croscarmellose sodium, crospovidone, sodium alginate, gums, starch, and magnesium aluminum silicate.

18. The pharmaceutical formulation according to claims 14 to 17, wherein the ratio by weight of the filler to the disintegrant is between 80:1 and 1:20, preferably between 50:1 and 1:10, and more preferably between 30:1 and 1:5.

19. The pharmaceutical formulation according to Claim 14, wherein said binders comprise polyvinylpyrrolidone (povidone), gelatin, sugars, glucose, natural gums, gums, synthetic celluloses, polymethacrylate, hydroxypropyl methylcellulose, hydroxypropyl cellulose, carboxymethyl cellulose, methyl cellulose and other cellulose derivatives, and the mixtures thereof.

20. The pharmaceutical formulation according to Claim 14, wherein said lubricants comprise sodium stearyl fumarate, magnesium stearate, polyethylene glycol, stearic acid, metal stearates, boric acid, sodium chloride benzoate and acetate, hydrogenated castor oil, sodium or magnesium lauryl sulfate, and the mixtures thereof.

21. The pharmaceutical formulation according to Claim 14, wherein said glidants comprise colloidal silicon dioxide, talk, aluminium silicate, and the mixtures thereof.

22. The pharmaceutical formulation according to Claim 14, wherein said sweeteners comprise aspartam, sucralose, saccharine; glucose, lactose, fructose and other sugars, and mannitol, sorbitol, xylitol, erythritol, and the mixtures thereof.

23. The pharmaceutical formulation according to Claim 14, wherein said aromatic agents comprise menthol, mint, cinnamon, chocolate, vanilin, and fruit extracts such as of cherry, orange, strawberry, grape, and the mixtures thereof.

24. The method for preparing a pharmaceutical formulation according to any of the preceding claims, comprising the following steps of:
a. mixing a part of all excipients except for the active agent in a container (mixture A), and the remaining part thereof in a separate container (mixture B),
b. mixing prasugrel free base with mixture A from the excipient mixtures obtained above in step (a) and then mixing the resulting mixture with mixture B,
c. converting the mixture obtained in step (b) into a suitable dosage form and packaging the same.

25. The method according to Claim 24, comprising the following steps of:
a. mixing the filler and the disintegrant among the excipients except for the active agent in a container (mixture A), and mixing the other excipients in a separate container (mixture B);
b. mixing prasugrel free base with mixture A from the excipient mixtures obtained above in step (a) and then mixing the resulting mixture with mixture B;
c. converting the resulting mixture into a suitable dosage form and packaging the same.
